# EUROPEAN PATENT SPECIFICATION

(11) **EP 2 033 597 B1**
(45) Date of publication and mention of the grant of the patent: **16.03.2011**
(21) Application number: 07115960.2
(22) Date of filing: 07.09.2007
(51) Int. Cl.: A61F 2/24

(54) **Fluid-filled delivery system for in situ deployment of cardiac valve prostheses**
Fluidgefülltes Freisetzungssystem für den in-situ-Einsatz von Herzklappenprothesen
Système de mise en place remplie de fluide pour le déploiement in situ de prothèses valvulaires cardiaques

(43) Date of publication of application: 11.03.2009
(73) Proprietor: SORIN BIOMEDICA CARDIO S.R.L., 13040 Saluggia (Vercelli) (IT); Mayo Foundation for Medical Education and Research, Rochester, MN 55905 (US)
(72) Inventor: RIGHINI, Giovanni, 10034, Chivasso (Torino) (IT); SURI, Rakesh Mark, Rochester, MN 55902 (US)
(74) Representative: Bosotti, Luciano

(56) References cited:
- WO-A-2006/054107
- FR-A- 2 828 091
- US-A1- 2002 117 264
- US-A1- 2006 241 656
- US-A1- 2007 162 103
- US-B1- 6 251 093

## Description

The present invention relates to instruments for the in situ delivery and positioning of implantable devices. Such instruments are known, for example, from WO 2006/054107 A2, FR 2 828 091 A1, US 2006/0241656 A1, US 2007/0162103 A1 and US 6,251,093. In particular, the invention relates to the in situ delivery of expandable prosthetic cardiac valves.

### BACKGROUND

There is much interest in the medical community in expandable prosthetic valves designed to be implanted using minimally-invasive surgical techniques (e.g., transthoracic microsurgery) or endovascular (i.e., percutaneous) techniques, which are less invasive than the surgical operations required for implanting traditional cardiac-valve prostheses. These expandable prosthetic valves typically include an anchoring structure or armature, which is able to support and fix the valve prosthesis in the implantation position, and prosthetic valve elements (e.g., leaflets or flaps), which are connected to the anchoring structure and are configured to regulate blood flow. One exemplary expandable prosthetic valve is disclosed in U.S. Publication 2006/0178740 A1.

Expandable prosthetic valves enable implantation using various minimally-invasive and/or sutureless techniques. Various techniques are generally known for implanting such an aortic valve prosthesis and include percutaneous implantation (e.g., transvascular delivery through a catheter), dissection of the ascending aorta using minimally-invasive thoracic access (e.g., mini-thoracotomy), and transapical delivery in which the aortic valve annulus is accessed directly through an opening in or near the apex of the left ventricle. There is a need in the art for improved systems for delivering the valve prosthesis to an appropriate location in a patient's cardiovascular system.

### SUMMARY

The present invention is a cardiac valve implantation system for implanting a cardiac valve prosthesis at an implantation site associated with a cardiac valve annulus as set for in the appended claims. The system includes an expandable cardiac valve prosthesis including a plurality of radially expandable portions and a plurality of valve leaflets. It further includes a deployment instrument including a control handle and a plurality of independently operable deployment elements operable to deploy the plurality of radially expandable portions. The system also includes a delivery device adapted to provide a pathway from outside the patient's body for delivery of the deployment instrument to the implantation site. The delivery device is adapted to provide a de-aired, fluid-filled, or gas-filled environment surrounding the prosthesis.

### BRIEF DESCRIPTION OF THE DRAWINGS

Figures 1 a and 1b illustrate, in general terms, the delivery instrument of the present invention, according to two exemplary embodiments.

Figure 2 is a partial cutaway, perspective view of a distal portion of the instrument of Figure 1, according to one embodiment of the present invention.

Figures 3a-3e illustrate a sequence of deploying a prosthetic heart valve using a retrograde approach.

Figures 4a-4e illustrate a sequence of deploying a prosthetic heart valve using an antegrade approach.

Figures 5a-5c illustrate a sequence of deploying a prosthetic heart valve.

Figures 6-9 illustrate further possible features of the instrument illustrated herein, according to various embodiments of the present invention.

Figure 10a-10d illustrate a sequence of deploying a prosthetic heart valve.

Figure 11 is a flowchart illustrating a method of implanting a valve prosthesis.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

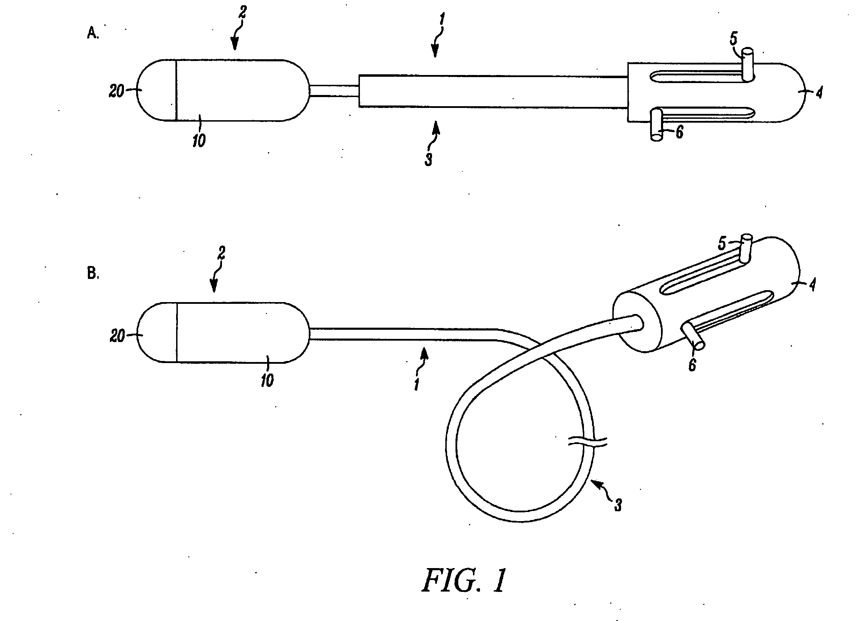

Figures 1a and 1b show an instrument 1 for implanting and radially deploying in situ an expandable, prosthetic cardiac valve. Purely by way of example, the prosthetic cardiac valve could be of the type described in U.S. Publication 2006/0178740 A1. As will be apparent to one skilled in the art, however, the instrument 1 could be used to deliver a variety of prosthetic cardiac valves including a plurality of radially expandable portions and is not limited to any particular prosthetic valve structure.

As shown in Figure 1, the instrument 1 includes a chamber or carrier portion 2 for enclosing and carrying the prosthetic device and a manipulation portion 3 that couples the carrier portion 2 to a control handle 4 where two actuator members (for instance two sliders 5, 6) are located. An optional third actuator is provided for that permits the carrier portion 2 to move forward, backward in relation to control handle 4. As will be appreciated, this feature permits for microadjustment of the carrier portion 2 and the valve prosthesis V in relation to a desired location while the control handle 4 is in a fixed location. A further optional actuator on the control handle 4 provides rotational adjustment of carrier portion 2 in relation to manipulation portion 3 and/or control handle 4. This permits the optional placement of the valve prosthesis through at least 360 degrees of rotation.

The manipulation portion 3 may assume various configurations. Figure 1a shows a configuration where the portion 3 is comprised of a substantially rigid bar with a length (e.g., 10 cm) that will permit positioning of the carrier portion 3, and the prosthetic cardiac valve carried thereby, at an aortic valve site. In various embodiments, instrument 1 is sized and dimensioned to permit easy surgical manipulation of the entire instrument as well as the actuators on the instrument without contacting the patient in a way to interfere with the positioning of the valve prosthesis V.

This configuration is adapted for use, for example, in the sutureless and the transapical implantation methods. Figure 1b, conversely, shows a second configuration, where the portion 3 is essentially comprised of an elongated, flexible catheter-like member that allows positioning of the carrier portion 3, and the prosthetic cardiac valve carried thereby, at an aortic valve site via transvascular catheterization (e.g., initiating at the femoral artery). This second configuration is also amenable for use in the sutureless or transapical implantation techniques. In one embodiment, the flexible, catheter-like member is braided or otherwise adapted to facilitate transmission of torque from the handle 4 to the carrier portion 2, such that the operator may effect radial positioning of the carrier portion 2 during the implantation procedure. Other features as described for the embodiment in Figure 1a may also be added to the embodiment in Fig. 1 b.

In one embodiment, the instrument 1 is adapted for use with a separate delivery tool. The instrument 1, for example, may be sized and shaped for delivery through a lumen of a tube or trocar during a "sutureless" or transapical delivery technique. Likewise, the instrument 1 may be adapted for delivery through a working lumen of a delivery or guide catheter. In this embodiment, for example, the operator may first deliver a guide catheter through the patient's vasculature to the implant site and then advance the instrument 1 through the lumen. According to another embodiment, the instrument 1 includes an axial lumen extending from a proximal end to a distal end. The lumen is sized to allow introduction and advancement of the instrument 1 over a previously-implanted guide wire. In other embodiments, other techniques known in the art are used to reach the implantation site from a location outside the patient's body.

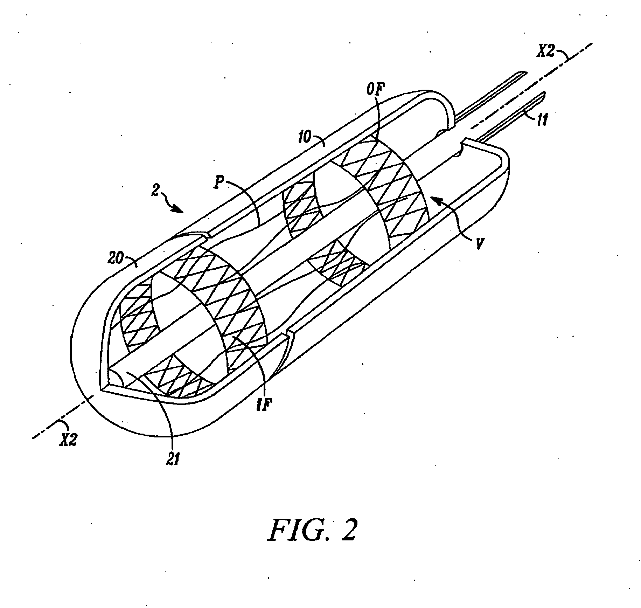

As shown in Figure 2, the carrier portion 2 includes two deployment elements 10, 20, each independently operable to allow the expansion of at least one corresponding, radially expandable portion of the implant device. In the case of the cardiac valve prosthesis, indicated as a whole as V, which is disclosed in U.S. Publication 2006/0178740 A1, two such radially expandable portions are provided situated respectively at the inflow end IF and the outflow end OF for the pulsated blood flow through the prosthesis. In alternative embodiments, however, the cardiac valve prosthesis may include more than two expandable members and, likewise, the carrier portion 2 may include more than two independent deployment elements. The valve prosthesis may be self-expanding (e.g., made from a superelastic material such as Nitinol) or may require expansion by another device (e.g., balloon expansion).

Figure 2 illustrates an embodiment for use with a self-expanding cardiac valve prosthesis. As shown in Figure 2, the cardiac valve prosthesis V is arranged within the carrier portion 2, such that an expandable portion IF and an expandable portion OF are each located within one of the deployment elements 10, 20. Each deployment element 10, 20 may be formed as a collar, cap or sheath. In yet a further embodiment the elements 10, 20 are porous (or have apertures) such that blood flow is facilitated prior, during and after placement of prosthesis V. As will be appreciated, blood flows through the elements 10, 20 and over or through the prosthesis V during the placement procedure. Each deployment element 10, 20 is able to constrain the portions IF, OF in a radially contracted position, against the elastic strength of its constituent material. The portions IF, OF are able to radially expand, as a result of their characteristics of superelasticity, only when released from the deployment element 10, 20. Typically, the release of the portions IF, OF is obtained by causing an axial movement of the deployment elements 10, 20 along the main axis X2 of the carrier portion 2. In one embodiment, the operator (e.g., physician) causes this axial movement by manipulating the sliders 5 and 6, which are coupled to the deployment elements 10, 20.

In yet an alternative embodiment, an optional micro-blood pump is operatively linked to the deployment elements 10 or 20 (or forms a part of the carrier portion 2, and serves to facilitate the movement of blood in a desired direction during the prosthesis placement procedure. The micro-blood pump can have a variable flow rate functionality to regulate blood flow as desired.

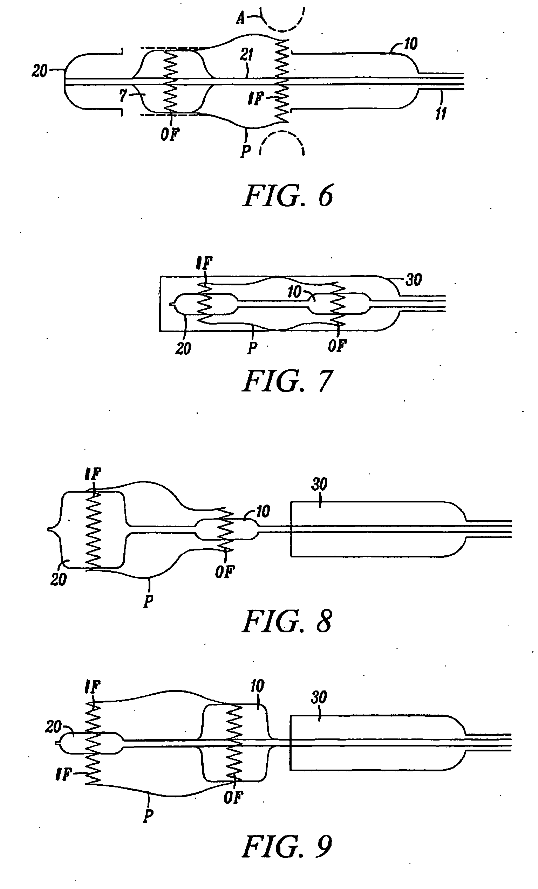

In an alternative embodiment (shown in Figures 7-9), expansion of the radially expandable portions IF, OF is caused by a positive expansion action exerted by the deployment elements 10, 20. In the embodiments shown in Figures 7-9, the deployment elements 10, 20 are comprised of expandable balloons onto which the portions IF, OF are coupled (e.g., "crimped") in a radially contracted position. In this embodiment, the operator causes radial expansion of the portions IF, OF by causing expansion of the balloons, using any of a variety of techniques.

The delivery instrument 1, including its various portions, is de-aired (i.e., atmospheric air is substantially removed), filled with a fluid (e.g. saline), or filled with an appropriate gas (e.g., carbon dioxide or nitrogen) prior to use. In one embodiment, the lumen of the delivery instrument, which may include a compressed prosthetic valve, is filled with a non-embolytic gas. This process eliminates the risk of an embolism occurring through the introduction of any of the portions described above directly into the beating heart of the patient. The delivery instrument, in particular the manipulator portion 3, includes an injection port adapted to mate with a syringe adapted for delivering a fluid or a gas to the delivery instrument. The injection port includes a seal for maintaining fluid pressure in the lumen and may include one or more pressure regulation devices.

According to an example not part of the present invention, a vacuum may be applied to the delivery instrument via a manifold at the proximal end of the manipulator portion 3. The applied vacuum ensures that all oxygen has been evacuated from the delivery tool. Additionally, the carrier portion 2 and the prosthetic valve therein may be assembled and sealed under vacuum.

According to yet another exemplary embodiment, the delivery instrument may be assembled in an environment saturated with a fluid, such as saline, or a gas, such as CO₂. For example, the carrier portion 2 and the prosthetic valve therein may be assembled in a saline or CO₂ saturated environment such that the carrier portion 2 is filled with the fluid, while the prosthetic valve is loaded into the chamber or carrier portion of the delivery instrument. According to various embodiments, the fluid includes a drug (e.g., heparin) selected to assist with the delivery of the prosthesis. The drug may, for example, include any drug known to manipulate or enhance cardiac function. According to other embodiments, the fluid includes an electrolytic solution.

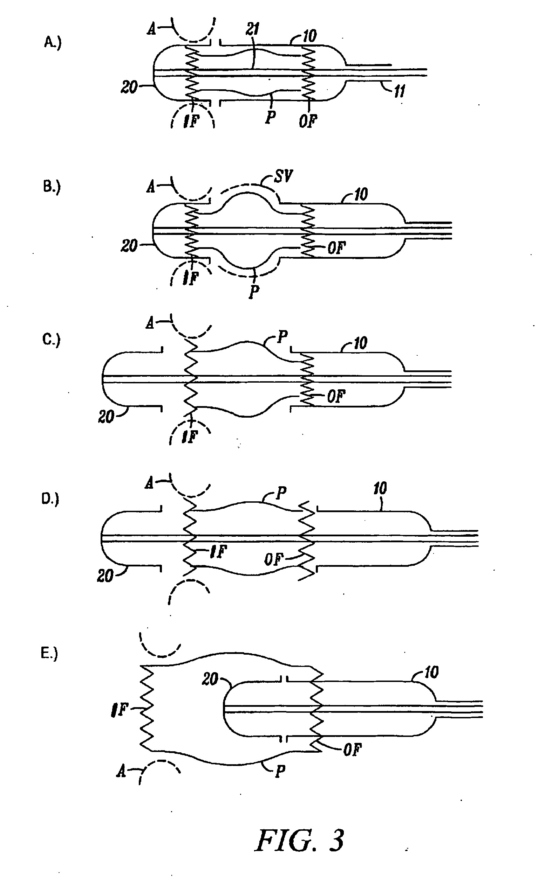

Figures 3-5 illustrate exemplary deployment techniques for wherein the expandable portions IF, OF are made of a self-expandable material. In Figures 3-5, only the armature of the prosthetic cardiac valve prosthesis V is schematically shown (i.e., the valve leaflets are not shown). As shown, the armature includes the expandable entry (inflow) portion IF and the expandable exit (outflow) portion OF, which are connected axially by anchoring formations P. In one embodiment, as described in U.S. Publication 2006/0178740, the formations P are spaced at 120° intervals about the armature circumference and are configured to radially protrude from the prosthesis V so as to penetrate into the sinuses of Valsalva.

In the case of a cardiac valve prosthesis to be deployed at an aortic position, the inflow end IF of the prosthesis V is located in correspondence with the aortic annulus, thereby facing the left ventricle. The profile of the aortic annulus is shown schematically by the dashed lines A in Figures 3-5. Conversely, the outflow end OF is located in the ascending line of the aorta, in a position immediately distal to the sinuses of Valsalva, wherein the formations P extend. The elements 10, 20 are
sized and dimensioned to accommodate anchoring elements of prosthetic valve V. This dimensioning can take the form in one embodiment of appropriate tailored protuberances that permit the anchoring elements to rest in an appropriately contracted position prior to positioning.

Figures 3-5 show a carrier portion 2 having two deployment elements 10, 20 each of which is capable of "encapsulating" (on one embodiment) or restraining a respective one of the inflow IF and outflow OF portions, to constrain the portions IF, OF from radially expanding. Both the elements 10, 20 can be arranged to slide longitudinally with respect to the principal axis X2 of the carrier portion 2. In yet another variant, the elements 10, 20 slide down and rotate around the principal axis X2 (e.g. in a cork screw fashion). The axial (and optional rotational) movement of the elements 10, 20 is obtained via the sliders 5, 6 provided on the handle 4 at the proximal end of the manipulation portion 3 of the instrument 1. For instance, the slider 5 may act on the deployment element 20 through a respective control wire or tendon 21, while the slider 6 may act on the deployment element 10 through a tubular control sheath 11 slidably arranged over the tendon 21, with both the sheath 11 and tendon 21 slidable along the axis X2.

In one exemplary embodiment, an internal surface of the elements 10, 20 comprise a low-friction or lubricious material, such as an ultra-high molecular weight material or PTFE (e.g., Teflon®). Such a coating will enable the elements 11, 21 to move or slide with respect to the portions IF, OF, such that the portions IF, OF are released upon axial movement of the elements 11, 21. In yet a further embodiment, other surfaces of the elements 10, 20 or any other parts of device 1 are coated or made from a low-friction material to provide for ease of insertion and manipulation within a subject.

In one embodiment, the sheath 11 is movable in a distal-to-proximal direction, so that the sheath and thus the element 10 move or slide "backwards" with respect to the carrier portion 2. In a complementary manner, the sliding movement of the tendon 21 will take place in a proximal-to-distal direction, so that the tendon and thus the element 20 move or slide "forward" with respect to the carrier portion 2. In another embodiment, movement of the elements 10, 20 is obtained by manipulating rigid actuation members from the handle 4.

In yet a further embodiment, While the device 1 is shown as being manually operable by a user, it is possible to have device 1 and the various positioning elements actuated by sensors (positional) and movement of the various elements of the device control by servo-motors, a microprocessor, and the like (e.g., computer controlled). It is appreciated that placement of the prosthesis V may be more precisely controlled through computer control and mechanical movement of the various elements of device.

Figures 3-5 are deliberately simplified for clarity of representation and do not take into account, for instance, the fact that the portion 3 of the instrument may include other control tendons/sheaths and/or ducts for inflating the post-expansion balloons (see Figure 6). Also, the element 20 could be actuated by means of a sheath rather than a tendon. Also, whatever their specific form of embodiment, the actuator members 11, 21 of the deployment elements 10, 20 may also have associated locking means (not shown, but of a known type) to prevent undesired actuation of the deployment elements 10, 20.

Notably, the deployment elements 10, 20 are actuatable entirely independently of each other. This gives the operator complete freedom in selecting which of the portions IF, OF to deploy first according to the specific implantation method or conditions. Figures 3a-3e, for example, illustrate use of the instrument 1 for a "retrograde" approach (e.g., in the case of sutureless or percutaneous implantation), to the valve annulus, wherein the cardiac valve prosthesis V approaches the valve annulus from the aortic arch.

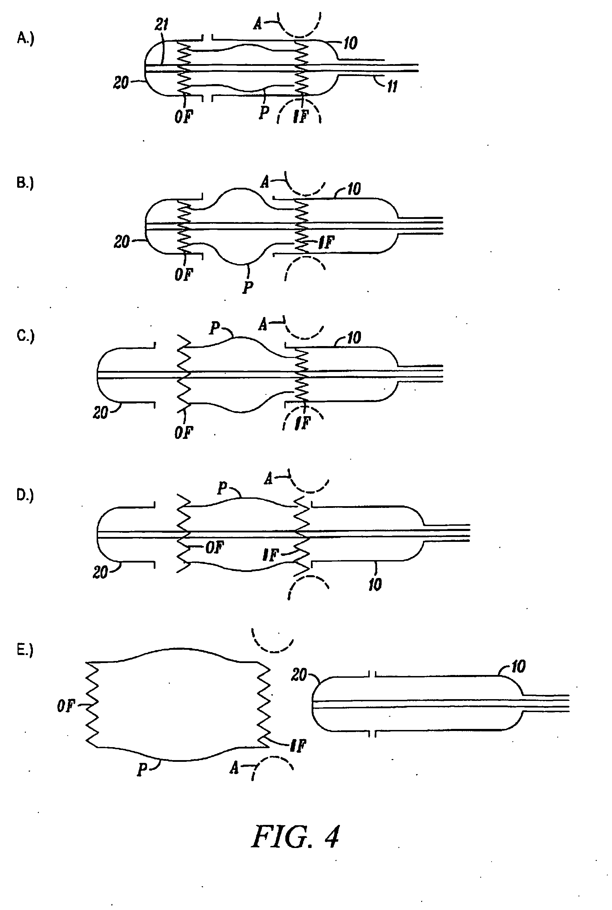

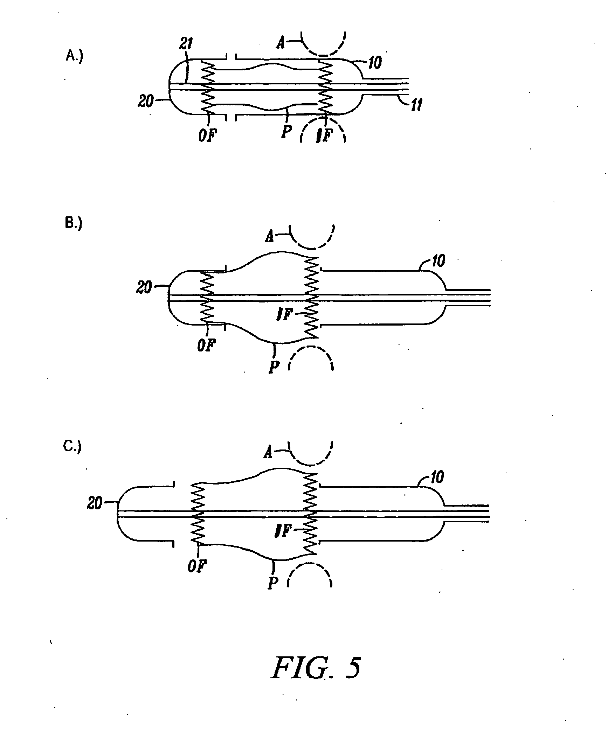

In Figure 3a (as in the following Figures 4a and 5a), the cardiac valve prosthesis V is shown mounted in or carried by the carrier portion 2 of the instrument 1, such that the deployment elements 10, 20 constrain the annular ends IF, OF of the prosthesis V in a radially contracted position.

Figure 3b shows the element 10 retracted axially with respect to the axis X2 of the carrier portion 2 a sufficient distance to uncover and release the formations P, which are then able to expand (e.g., due to their superelastic construction) such that they protrude beyond the diameter of the elements 10, 20. As shown in Figure 3b, the formations P are allowed to expand, while the remaining portions of the prosthesis V are maintained in a radially contracted configuration. In the configuration shown in Figure 3b, the operator can take the necessary action for ensuring the appropriate positioning of the prosthesis V in correspondence with the sinuses of Valsalva SV. The profile of the sinuses of Valsalva are shown schematically in Figure 3b by the dashed lines SV. Prosthesis V has elements sized and dimensioned to completely conform to the sinuses Valsalva in one variant of the invention.

Such appropriate positioning includes both axial positioning (i.e. avoiding deploying the prosthetic valve V too far "upstream" or too far "downstream" of the desired position with the ensuing negative effect that the inflow end IF is not correctly positioned with respect to the valve annulus A) and radial positioning. The sinuses of Valsalva are configured as a hollow, three-lobed structure. Accordingly, accurately positioning each formation P of the prosthesis V in a respective sinus of Valsalva will ensure the correct positioning or angular orientation of the prosthetic valve as a whole, which will ensure that the leaflets of the prosthetic valve are correctly oriented (i.e., extend at the angular positions of the annulus where the natural valve leaflets were located before removal).

In exemplary embodiments the instrument 1 may further include various structures or features to assist the operator in obtaining the appropriate axial positioning with respect to the aortic annulus and radial positioning with respect to the sinuses of Valsalva. The instrument 1 (or the guide catheter or delivery tube), for example may include a lumen sufficient to allow the injection of contrast fluid to a location at the implantation site. For the embodiment shown in Figure 3, for example, this lumen would have an opening located past the inflow end IF or the prosthesis V, such that any injected contrast fluid would then flow back toward the prosthesis V, thereby enabling the operator to obtain a visual image of the implantation site, including an image of the sinuses of Valsalva. Likewise, the prosthesis V may include radiopaque markers disposed at appropriate locations to assist in this positioning.

In one example (e.g., in the case of "sutureless" implantation), the carrier portion 2 and the prosthesis V may be arranged from the beginning in the configuration represented in Figure 3b, namely with the formations P already protruding radially with respect to the profile of the prosthesis, while the annular end portions IF, OF are constrained in a radially contracted position by the elements 10, 20. In this case, the element 10 will have a sufficient length only to cover the axial extension of the annular end portion OF, as it need not radially constraint the formations P.

Figure 3c shows the element 20 displaced distally with respect to the prosthesis V by the tendon 21. As shown, the element 20 was displaced a length sufficient to uncover the annular inflow portion IF, such that the portion IF is able to expand radially to assume the desired anchoring position at the valve annulus A. This release of the inflow portion IF takes place while the prosthetic valve V is still precisely retained and controlled by the instrument 1, such that it will not move or "jump" with respect to the valve annulus during the expansion of the portion IF.

It will also be appreciated that from the configuration shown in Figure 3c, the operator may return to the configuration shown in Figure 3a, so as to cause a radial contraction of the formations P and, even if in an incomplete manner, of the annular inflow portion IF. This will allow the operator to withdraw the prosthesis V from the implantation site if the operator believes that the implantation procedure has thus far not yielded a satisfactory result. In this variant, a re-contracting element is provided as part of device 1 that permits either the outflow OF and/or inflow IF portion to be re-constricted. By way of example a cutable thread could be used to re-crimp either portion.

Next, the prosthetic implantation process progresses by sliding the deployment element 10 so that it releases the outflow annular portion OF. The portion OF can then radially expand against the aortic wall, thus completing the second phase of the implantation operation of the prosthesis V.

Finally, as shown in Figure 3e, the carrier portion 2 and the instrument 1 as a whole can be withdrawn with respect to the implantation site through the center of the prosthesis V. In one embodiment, the carrier portion 2 is withdrawn after the deployment elements 10, 20 have been brought back to their initial positions, that is after having caused the elements 10, 20 to slide, in a proximal-to-distal and in a distal-to-proximal direction, respectively. The sequence of operations represented in Figures 3a-3e may be accomplished with a pulsating heart and without interrupting the natural circulation of blood.

The instrument 1 is adapted such that substantially oxygen-free environment can be maintained through out the valve implantation procedure. According to one embodiment, the instrument includes a seal or one or more pressure sensors and/or regulators for maintaining a constant flow a fluid, such as saline, or a constant pressure of CO₂ at the implantation site through the delivery instrument 1 in order to minimize and/or prevent air embolism during the procedure.

Figures 4a-4e show an implantation procedure of a prosthesis V. This procedure is similar to the procedure shown in Figures 3a-3e, but Figures 4a-4e show an "antegrade" approach, typical of a transapical implantation procedure. In this case, the prosthesis V (mounted in the carrier portion 2) is advanced to the implantation site (e.g., aortic valve) through the left ventricle. While reference is again made herein to a prosthetic valve for the substitution of the aortic valve, it will be once more appreciated that the same criteria and principles will also apply to different valve types (e.g. mitral). Various techniques for accessing the aortic valve site through the left ventricle are known in the art. One exemplary technique for transapical delivery is disclosed in U.S. Publication 2005/0240200.

Figures 4a-4e are substantially identical to Figures 3a-3e, except that the position assumed by the prosthetic valve V is inverted. Accordingly, in the case of the intervention of "antegrade" type of Figures 4a-4e, the carrier portion 2 of the instrument 1 with the prosthesis V mounted therein is passed completely through the valve annulus A, so as to position the inflow portion IF in correspondence with the valve annulus A. As it is appreciated the device 1 is configured and sized and dimensioned so that it is capable of delivering the prosthesis V in both an antegrade and a retrograde intervention.

After withdrawing the deployment element 10, so as to release the formations P (Figure 4b), the deployment element 20 is advanced distally, so as to release and allow the outflow annular end portion OF to radially expand against the aortic wall downstream of the sinuses of Valsalva (see Figure 4c). At this point, the operator is still in a position to ensure that the prosthesis has the required correct angular position by making sure that the formations P each correctly engage a corresponding sinus. If the formations P do not properly align with the sinuses of Valsalva, the operator may use the instrument to apply a torque to the prosthesis V, thereby causing a rotation of the prosthesis V into the proper angular position. In one exemplary embodiment, the tendon 21 includes a stop (not shown) configured to prohibit axial motion of the inflow portion IF. This stop may help prevent axial movement of the inflow portion IF during distal motion of the of the deployment element 20, thereby ensuring that the outflow portion OF is released before the inflow portion IF.

Subsequently, by completely withdrawing in a proximal direction the deployment element 10, the operator releases the annular inflow portion IF that is thus deployed in correspondence with the aortic valve annulus thus completing the two-step implantation procedure of the prosthetic valve V (see Figure 4d). Then, the procedure progresses by bringing the deployment elements 10, 20 back towards their initial position with the ensuing retraction of the instrument 1 from the inflow portion IF of the valve (Figure 4e).

Figures 5a-5c, which correspond closely to the sequence of Figures 4a-4c, show that (also for a procedure of the "antegrade" type) it is possible to effect the two-step implantation sequence of Figures 4a-4e by deploying the end portions IF and OF of the prosthetic valve V in the reverse order. In the technique of Figures 5a-5c, once the desired "axial" position is reached (as represented in Figure 5a, which is practically identical to Figure 4a) with the expandable inflow end IF in correspondence of the aortic valve annulus A, the inflow portion IF is expanded first by operating the deployment element 10 to release the corresponding inflow portion IF.

The implantation procedure then proceeds, as schematically represented in Figure 5c, with the second step of this two-step procedure, namely with the deployment element 20 advanced distally with respect to the prosthesis V so as to release the expandable outflow portion OF. The outflow portion OF is thus free to expand against the aortic wall in a region downstream of the sinuses of Valsalva into which the formations P protrude.

The teaching provided in Figures 5a-5c also apply in the case of a "retrograde" procedure, as shown in Figures 3a-3e. Because the deployment elements 10, 20 are adapted to be activated entirely independently of each other, the operator is free to choose the most suitable deployment sequence (inflow first and then outflow; outflow first and then inflow) as a function of the specific conditions of intervention. This sequence may be entirely independent of access to the implantation site being of the retrograde or antegrade type.

Figures 6 and 7 schematically illustrate embodiments in which the carrier portion 2 of the instrument 1 includes a balloon 7 at locations corresponding to at least one or to both annular ends of the cardiac valve prosthesis V. This balloon may be of any known type (e.g. of the type currently used in expanding stents or the like in a body lumen, which therefore does require a detailed description to be provided herein) and is intended for use in realizing a "post-expansion" of the corresponding end portion IF, OF of the prosthesis V, so as to radially urge it against the wall of the implantation lumen. For instance, as shown in Figure 6, the balloon 7 can be selectively expanded (by inflating it with well known means and criteria) in such a way as to produce a radial expansion of the expandable portion associated therewith (here the end portion OF).

This technique may be useful to avoid movement or "jumping" of the prosthesis V during implantation. For instance, if the operator fears that deployment of the inflow end portion IF in correspondence of the aortic annulus A may give rise to an undesired longitudinal displacement of the valve prosthesis V as a whole, while the inflow portion IF is being released by the element 10 and expands to engage the aortic annulus A, a post-expansion balloon 7 associated with the outflow end OF can be inflated. In this way, as long as the post-expansion balloon 7 is kept dilated, the outflow end OF is urged and thus safely anchored to the lumen wall and any undesired displacement of the prosthetic valve V in an axial direction is prevented. Once the inflow portion IF is safely positioned at the aortic annulus A, the balloon 7 can be deflated and the instrument 1 withdrawn.

Figures 7, 8 and 9 schematically illustrate, without the intent of making any specific distinctions between "antegrade" and "retrograde" approaches and any specific choice as to which end portion, inflow IF or outflow OF, is to be deployed first, that the same two-step mechanism for independently deploying the two end portions IF, OF illustrated in Figures 3, 4 and 5 can be implemented in the case of prostheses V including end portions IF, OF whose radial expansion is obtained via a positive outward expansion action exerted by means of deployment elements 10, 20 altogether constituted by expandable balloons. These may be balloons of any known type and substantially correspond, from a structural point of view, to the post-expansion balloons (see for instance the balloon 7 of Figure 6).

Other embodiments of the present invention include "hybrid" solutions, where a cardiac valve prosthesis V includes one or more self-expandable portions (having associated deployment elements 10, 20 of the type illustrated in Figures 2-5) as well as one or more portions radially expandable via an expandable deployment element (such as a balloon as illustrated in Figures 7-9).

In one variant and in the case where expansion due to a positive action of one or more balloons is preferred over the use of a self-expandable portion, the same balloon may be used both as an expansion balloon (Figures 7, 8 and 9), and as a post-expansion balloon (Figure 6).

As schematically illustrated in Figures 7-9 (the same solution can be adopted also in the case of Figures 2-6, it is possible to provide a tubular sheath 30 that surrounds in the manner of a protective tunic the assembly comprised of the carrier portion 2 with the prosthetic valve V mounted therein. This with the purpose of facilitating, typically in a percutaneous implantation procedure, the advancement towards the implantation site through the tortuous paths of the vasculature of the patient without risks of undesired jamming or kinking. It will be appreciated that, for the same goal, the deployment elements 10, 20 normally exhibit a "streamlined" shape, exempt from protruding parts and/or sharp edges. This is particularly the case for the element 20 located at a distal position, which typically exhibits an ogive-like shape.

In yet another variant of the invention, the elements 10, 20 are formed of a material that permits them to collapse after deployment of the prosthesis V. It is appreciated that this permits the entire device to be more easily removed from the subject.

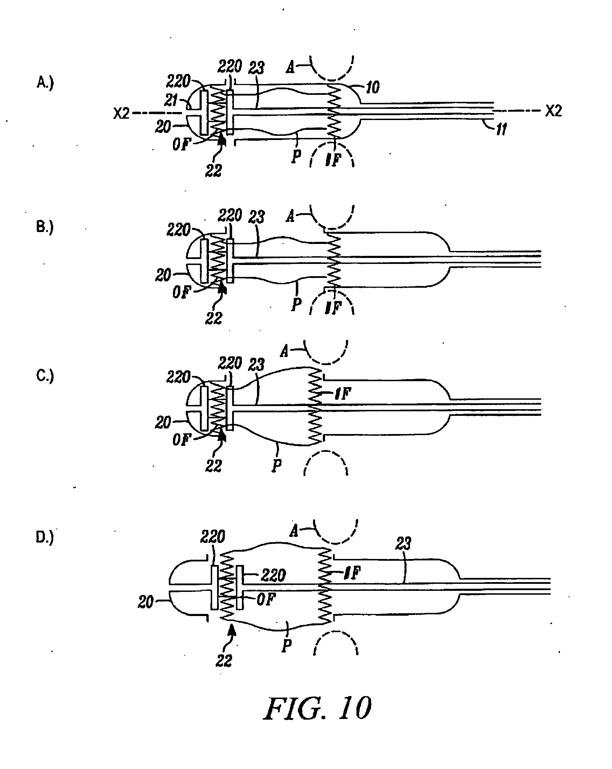

Figures 10a-10d, which substantially correspond to Figures 5a-5c, illustrate an embodiment associating with either or both of the annular end portions IF, OF of the prosthesis V and "anti-skid" locking member 22. This member is primarily intended to prevent any undesired sliding movement of the end portion (IF and/or OF) with respect to its deployment element lengthwise of the carrier portion 2. Such a locking member is preferably associated with (at least) the annular end portion to be deployed second in the two-step deployment process of the prosthetic valve V described herein.

In this exemplary embodiment, the locking member 22 takes the form of a hub positioned at the distal end of a tubular member 23 having the wire 21 slidably arranged therein. The sheath 11 surrounds the tubular member 23 and is adapted to slide thereon so that the locking member 22 is capable of maintaining at a fixed axial position (e.g. via end flanges 220) the annular outflow portion OF with which the locking member is associated. The annular end portion in question is thus prevented from sliding axially of the deployment element 20, at least as long as the annular end portion OF is radially constrained by the deployment element 20.

The arrangement described makes it possible to adjust the position of the annular end portion locked by the locking member (and the position of the valve prosthesis V as a whole) both axially and angularly to the implantation site. This applies more or less until the annular portion expands to the point where further displacement is prevented by engagement of the annular portion with the valve annulus or the aortic wall. Additionally, the presence of the locking member(s) 22 facilitates possible recovery of the prosthetic valve V in case the implantation procedure is to be aborted.

According to various embodiments of the present invention, the delivery system is capable of both antegrade and retrograde delivery of instruments and the like. One exemplary embodiment of such a delivery system is disclosed in co-pending, commonly assigned U.S. patent application number 11/851523, filed on even date herewith, entitled "Prosthetic Valve Delivery System Including Retrograde/Antegrade Approach".

## Claims

1. A prosthetic valve delivery system for implanting a cardiac valve prosthesis (V) at an implantation site associated with a cardiac valve annulus (A), the system comprising an expandable cardiac valve prosthesis (V) including a plurality of radially expandable portions (IF, OF) and a deployment instrument (1) including a chamber (2) for holding the valve prosthesis (V), the system being **characterized in that** the chamber (2) is filled with a fluid, gas or combination thereof **and in that** the deployment instrument (1) includes a lumen and an injection port adapted to mate with a syringe adapted for delivering a fluid or a gas or a combination thereof to the deployment instrument, wherein the injection port includes a seal for maintaining fluid pressure in the lumen.

2. The system of Claim 1, wherein the deployment instrument (1) includes a manipulator portion (3) and a manifold at a proximal end of said manipulator portion (3)

3. The system of claim 1 wherein the cardiac valve prosthesis (V) is an aortic valve prosthesis and further wherein the chamber (2) includes a plurality of operable deployment elements (10, 20).

4. The system of claim 3 wherein the plurality of radially expandable portions (IF, OF) include an annular inflow portion (IF), associated with blood inflow, and an annular outflow portion (OF), associated with blood outflow.

5. The system of claim 1 wherein the gas is selected from a non-embolytic gas.

6. The system of claim 1 wherein the gas is selected from carbon dioxide and nitrogen.

7. The system of claim 1 wherein the fluid further comprises a drug.

8. The system of claim 7 wherein the drug is selected from the group consisting of heparin, a drug to manipulate cardiac function to assist with the delivery of the prosthesis (V).

9. The system of claim 1 in which the fluid comprises an electrolytic solution.

## Patentansprüche

1. Ein Klappenprothesen-Freisetzungssystem zum Implantieren einer Herzklappenprothese (V) an einem Implantationsort, dem ein Herzklappenring (A) zugeordnet ist, wobei das System eine expandierbare Herzklappenprothese (V) mit einer Mehrzahl von radial expandierbaren Bereichen (IF, OF) und ein Freisetzungsinstrument (1) mit einer Kammer (2) zum Halten der Klappenprothese (V) aufweist, wobei das System **dadurch gekennzeichnet ist, dass** die Kammer (2) mit einer Flüssigkeit, einem Gas oder einer Kombination daraus gefüllt ist und dass das Freisetzungsinstrument (1) ein Lumen und eine Einspritzöffnung aufweist, die geeignet ist, mit einer Spritze zusammenzupassen, die geeignet ist, dem Freisetzungsinstrument ein Fluid oder Gas oder eine Kombination daraus zuzuführen, aufweist, wobei die Einspritzöffnung eine Dichtung aufweist, um einen Fluiddruck in dem Lumen beizuhalten.

2. Das System von Anspruch 1, worin das Freisetzungsinstrument (1) im Betätigungsbereich (3) und einen Verteiler an dem poximalen Ende des Betätigungsbereichs (3) aufweist.

3. Das System von Anspruch 1, worin die Herzklappenprothese (V) eine aortische Klappenprothese ist und worin weiterhin die Kammer (2) eine Mehrzahl von betätigbaren Freisetzungselementen (10, 20) umfasst.

4. Das System von Anspruch 3, worin die Mehrzahl von radial expandierbaren Bereichen (IF, OF) einen ringförmigen Einströmbereich (IF), der einströmendem Blut zugeordnet ist, und einen ringförmigen Ausströmbereich (OF), der einem ausströmenden Blut zugeordnet ist, umfasst.

5. Das System von Anspruch 1, worin das Gas aus einem nicht embolytischen Gas ausgewählt ist.

6. Das System von Anspruch 1, worin das Gas aus Kohlendioxid und Stickstoff ausgewählt ist.

7. Das System von Anspruch 1, worin das Fluid weiterhin ein Medikament enthält.

8. Das System von Anspruch 7, worin das Medikament ausgewählt ist aus der Gruppe bestehend aus Heparin, einem Medikament zum Manipulieren der Herzfunktion, um die Freisetzung der Prothese (V) zu unterstützen.

9. Das System von Anspruch 1, in dem das Fluid eine elektrische Lösung umfasst.

## Revendications

1. Système de pose de valvule prothétique pour implanter une prothèse de valvule cardiaque (V) au niveau d'un site d'implantation associé à un anneau de valvule cardiaque (A), le système comprenant une prothèse de valvule cardiaque (V) expansible comprenant une pluralité de parties radialement expansibles (IF, OF) et un instrument de déploiement (1) comprenant une chambre (2) pour contenir la prothèse valvulaire (V), le système étant **caractérisé en ce que** la chambre (2) est remplie avec un fluide, un gaz ou leur combinaison et **en ce que** l'instrument de déploiement (1) comprend une lumière et un orifice d'injection adapté pour se coupler avec une seringue adaptée pour fournir un fluide ou un gaz ou leur combinaison à l'instrument de déploiement, dans lequel l'orifice d'injection comprend un joint d'étanchéité pour maintenir la pression de fluide dans la lumière.

2. Système selon la revendication 1, dans lequel l'instrument de déploiement (1) comprend une partie de manipulateur (3) et un collecteur au niveau de l'extrémité proximale de ladite partie de manipulateur (3).

3. Système selon la revendication 1, dans lequel la prothèse de valvule cardiaque (V) est une prothèse de valvule aortique et en outre dans lequel la chambre (2) comprend une pluralité d'éléments de déploiement (10, 20) opérationnels.

4. Système selon la revendication 3, dans lequel la pluralité de parties radialement expansibles (IF, OF) comprend une partie d'écoulement entrant annulaire (IF), associée à l'écoulement entrant du sang, et une partie d'écoulement sortant annulaire (OF) associée à l'écoulement sortant du sang.

5. Système selon la revendication 1, dans lequel le gaz est sélectionné à partir d'un gaz non embolique.

6. Système selon la revendication 1, dans lequel le gaz est choisi d'entre le dioxyde de carbone et l'azote.

7. Système selon la revendication 1, dans lequel le fluide comprend en outre un médicament.

8. Système selon la revendication 7, dans lequel le médicament est choisi dans le groupe comprenant l'héparine, un médicament pour manipuler la fonction cardiaque afin d'aider la pose de la prothèse (V).

9. Système selon la revendication 1, dans lequel le fluide comprend une solution électrolytique.
